# EUROPÄISCHE PATENTSCHRIFT

(11) **EP 1 518 504 B1**
(45) Veröffentlichungstag und Bekanntmachung des Hinweises auf die Patenterteilung: **03.11.2010**
(21) Anmeldenummer: 04018246.1
(22) Anmeldetag: 02.08.2004
(51) Int. Cl.: A61B 17/17

(54) **Zielgerät für einen Verriegelungsnagel**
Guide device for a locking nail
Guide de visée pour clou de verrouillage

(30) Priorität: 24.09.2003 DE 20314742 U
(43) Veröffentlichungstag der Anmeldung: 30.03.2005
(73) Patentinhaber: Stryker Trauma GmbH, 24232 Schönkirchen (DE)
(72) Erfinder: Simon, Bernd, 26106 Kiel (DE); Füllgraf, Rene, 24239 Achterwehr (DE)
(74) Vertreter: Kopf, Korbinian Paul

(56) Entgegenhaltungen:
- EP-A- 1 382 308
- WO-A-97/13467
- US-A- 5 411 503
- US-A- 5 584 838
- US-A1- 2003 139 669
- US-B1- 6 261 247

## Beschreibung

Die folgende Erfindung betrifft ein Zielgerät für einen Verriegelungsnagel, der zwei entlang seiner Längsrichtung angeordnete Schwingkreise besitzt.

In den Unterschenkel- oder Oberschenkelknochen einführbare Verriegelungsnägel weisen zumeist mehrere Querbohrungen auf, durch die Knochenschrauben geführt werden, um den Verriegelungsnagel sicher im Knochenkanal zu halten. Eine besondere Schwierigkeit hierbei ist das Auffinden der Querbohrung beim implantierten Nagel. Hierfür werden Zielgeräte eingesetzt. Bei einer Kategorie von Zielgeräten wird der Knochen mit Röntgenstrahlen durchleuchtet und die Querbohrungen des Verriegelungsnagels in dem Knochen auf einem Monitor sichtbar gemacht. Zusätzlich wird ein Zielelement in dem Röntgenbild dargestellt, so daß an der Außenseite des Knochens eine auf der Achse der Querbohrung liegende Markierung vorgenommen werden kann.

Bei einer anderen Kategorie von Zielgeräten ist dies fest mit dem implantierbaren Nagel verbunden. Ein bügelartiger Abschnitt weist mindestens eine Bohrung auf, deren Achse mit der Achse der Querbohrung des Nagels ausgerichtet ist, wenn das Zielgerät angebracht ist. Zur Führung des Bohrwerkzeugs bzw. der Knochenschraube ist auch bekannt, durch die Bohrung des Zielgeräts eine Führungshülse zu stecken, die bis gegen die Außenseite des Knochens vorgeschoben wird.

Aus US 5,411,503 ist ein Instrument zum Zielen für Verriegelungsnägel in Markknochen bekannt geworden. Bei dem Verfahren wird ein Spieß in den hohlen Schaft des Verriegelungsnagels eingeführt. Der Spieß ist an seinem distalen Ende mit zwei Schwingkreisen versehen. An dem proximalen Ende weist der Spieß einen Anschlag auf. Zur Positionierung wird der Spieß vollständig in den eingetriebenen Verriegelungsnagel eingesetzt, wobei das Anschlagelement an dem freien Ende des Verriegelungsnagels anliegt. Die beiden Schwingkreise am Ende des Spießes werden angeregt und das abgestrahlte elektromagnetische Feld durch entsprechende Sensoren in dem Zielgerät erfaßt. Das Zielgerät wird in dem Feld der Schwingkreise derart ausgerichtet, daß ein mit diesem verbundener Führungskanal für den Bohrer entsprechend der Achse der Querbohrung an dem Knochennagel ausgerichtet ist. Nachteilig an dieser Vorrichtung ist, daß eine während des Eintreibens auftretende Verwindung oder Stauchung des Verriegelungsnagels nicht berücksichtigt werden kann.

Aus US 5,584,838 ist ein Knochennagel bekannt, in dessen hohlen Schaft ein Element mit einem einzelnen Schwingkreis eingesetzt ist. Der Nagel wird mit eingesetztem Element in den Knochen eingetrieben, so daß die vorstehend beschriebenen Nachteile bei einer Verwindung des Nagels nicht auftreten können. Nachteilig an einem derart ausgebildeten Verriegelungsnagel ist jedoch, daß durch die Verwendung nur eines Schwingkreises ein vergleichsweise aufwendiges Zielgerät erforderlich ist, um den Bohrer auszurichten. Ferner erweist es sich als nachteilig, daß der Schwingkreis genau in dem Kanal der Querbohrung angeordnet ist und nach einer Ausrichtung des Zielgeräts entfernt werden muß. Ein Zielgerät gemäss dem Oberbegriff von Anspruch 1 ist ebenfalls offenbart.

Aus DE 33 32 642 ist eine weitere Vorrichtung zum Auffinden von Querbohrungen in intramedulären Implantaten bekannt. Hierbei wird ein Magnetfeld in der Querbohrung ausgerichtet und mit einer Magnetfeldmeßeinrichtung von außen die Achse der Querbohrung gemessen.

Aus EP 0 523 905 ist ein Zielgerät zur axialen Ausrichtung einer Führungshülse bekannt, bei dem die Führungshülsen mit zwei Paaren von Meßspulen versehen sind, die das Magnetfeld einer in dem Nagel angeordneten und mit der Querbohrung ausgerichteten Spule erfassen. Zur Ausrichtung wird das Zielgerät so positioniert, daß jedes Paar der zwei Spulen ein gleich starkes Magnetfeld empfängt.

Der vorliegenden Erfindung liegt die Aufgabe zugrunde, ein Zielgerät für einen Verriegelungsnagel bereitzustellen, bei dem mit einfachen Mitteln möglichst genau und zuverlässig die Position der Querbohrung in dem Verriegelungsnagel erfaßt werden kann.

Erfindungsgemäß wird die Aufgabe durch ein Zielgerät mit den Merkmalen aus Anspruch 1 gelöst. Vorteilhafte Ausgestaltungen bilden den Gegenstand der Unteransprüche 2 bis 13.

Das erfindungsgemäße Zielgerät ist geeignet, eine Führungshülse zum Ansetzen eines Bohrers und/oder einer Knochenschraube relativ zu einer Querbohrung eines eingesetzten Verriegelungsnagels auszurichten. Der Verriegelungsnagel besitzt hierzu zwei entlang der Längsrichtung des Verriegelungsnagels angeordnete Schwingkreise. Im ausgerichteten Zustand des Zielgeräts fällt die Mittelachse der Führungshülse im wesentlichen mit der Mittelachse der Querbohrung zusammen. Das Zielgerät besitzt in einer ersten Ebene drei oder mehr Schwingkreise, die von einem in der Ebene liegenden ersten Zentrum jeweils den gleichen Abstand besitzen. In einer zweiten parallel zu der ersten Ebene verlaufenden Ebene sind ebenfalls drei oder mehr Schwingkreise angeordnet, die einen gleichen Abstand von einem in der zweiten Ebene liegenden Zentrum besitzen. Erste und zweite Ebene sind so zueinander angeordnet, daß durch das erste und das zweite Zentrum eine Flächennormale zu einer der Ebenen verläuft. Zusätzlich ist ein Paar von Schwingkreisen beabstandet von der durch die Zentren verlaufenden Geraden vorgesehen, wobei die Verbindungsgerade der Schwingkreise in dem mit dem Verriegelungsnagel ausgerichteten Zustand des Zielgeräts im wesentlichen senkrecht zur Längsrichtung des Verriegelungsnagels steht. Die erste Ebene dient zur Positionierung in einer xy-Ebene. Zur besseren Übersicht verläuft die Achse der Querbohrung in z-Richtung und die Längsrichtung des Verriegelungsnagels in x-Richtung. Selbstverständlich kann das erfindungsgemäße Zielgerät auch eingesetzt werden, wenn Längsrichtung des Verriegelungsnagels und Achse der Querbohrung nicht orthogonal zueinander stehen. Bei dieser Geometrie definiert die erste Ebene mit den Schwingkreisen die Position des Zielgeräts in der xy-Ebene. Zur Auffindung der richtigen Position wird die Position des Zielgeräts gesucht, in dem die Schwingkreise in der ersten Ebene ein elektromagnetisches Feld eines Schwingkreises in dem Verriegelungsnagel mit gleicher Stärke empfangen. Ist die Position in der xy-Ebene festgelegt, so muß sichergestellt werden, daß die Achse der Führungshülse oder der Zieleinrichtung nicht gegenüber der z-Achse bzw. der Achse der Querbohrung verkippt ist. Hierzu dient die zweite, parallel zur ersten Ebene angeordnete Ebene mit ihren Schwingkreisen. Werden auch von diesen Schwingkreisen Signale gleicher Stärke erfaßt, so ist sichergestellt, daß die Achsen von Führungshülse und Querbohrung zusammenfallen. Allerdings kann hierbei das Zielgerät noch um diese Achse gedreht werden. Um auch diesen Freiheitsgrad festzulegen, wird mit dem zusätzlichen Paar von Schwingkreisen das Signal eines zweiten Schwingkreises aus dem Vernegelungsnagel gemessen. Hierzu wird das zusätzliche Paar von Schwingkreisen senkrecht zu der Längsrichtung des Verriegelungsnagels ausgerichtet. Empfangen beide Schwingkreise Signale gleicher Stärke so ist das Zielgerät vollständig in einer genau definierten Position zu dem Knochennagel ausgerichtet und die Lage der Querbohrungen kann relativ zu dieser Referenzposition bestimmt werden. Die Spulen aus zusätzlichen Spulenpaar sind in "y"-Richtung ausgerichtet und befinden sich somit auf beiden Seiten der in Längsrichtung verlaufenden "x"-Richtung. Die in dem Verriegelungsnagel und in dem Zielgerät vorgesehenen Schwingkreise werden vorzugsweise mit ihrer Resonanzfrequenz betrieben.

Damit die Aufnahme-Schwingkreise ein elektromagnetisches Signal empfangen können und entsprechend dieser Anregung schwingen, ist es erforderlich, daß die Schwingkreise in dem Verriegelungsnagel angeregt werden. Erfindungsgemäß wird zur Anregung dieser Schwingkreise eine Anregungsspule in dem Zielgerät vorgesehen, die einen Hochfrequenzimpuls zur Anregung der Schwingkreise in den Verriegelungsnagel abstrahlt.

In einer bevorzugten Ausgestaltung sind in der ersten und/oder zweiten Ebene vier Schwingkreise in einem Rechteck zueinander angeordnet. Bevorzugt wird als Rechteck ein Quadrat gewählt. Durch die rechteckige Anordnung der Aufnahme-Schwingkreise in den Ebenen des Zielgeräts wird die Genauigkeit bei der Positionierung deutlich erhöht.

In einer bevorzugten Ausgestaltung sind die Schwingkreise in der ersten und zweiten Ebene deckungsgleich zueinander angeordnet. Dies bedeutet, daß, wenn die parallel zueinander verlaufenden Ebenen entlang ihrer Flächennormalen zur Deckung gebracht werden, die Schwingkreise in den Ebenen übereinander liegen.

In einer möglichen Ausgestaltung des erfindungsgemäßen Zielgeräts können die von den Schwingkreisen empfangenen Signale an eine interne oder externe Auswerteeinheit weitergeleitet werden. Zur Anzeige der richtigen Position ist es möglich, daß dies an dem Zielgerät selbst oder an einer externen Anzeige oder an beidem erfolgt.

Die Auswerteeinheit besitzt Filter- und Verstärkungseinheiten, die auf eine Arbeitsfrequenz der Schwingkreise abgestimmt sind. Die Verstärkereinheit weist bevorzugt einen Verstärker und einen Bandpaß auf, wobei die Verstärkung im wesentlichen durch einen aktiven Bandpaß erfolgt.

Die verstärkten und gefilterten Meßwerte werden gleichgerichtet und über einen A-D-Wandler digitalisiert. Die digitalisierten Daten können über eine serielle Schnittstelle an einem PC zur Auswertung weitergeleitet werden. An dem PC werden die zusätzlich oder ausschließlich ausgewerteten Daten dargestellt, um den ausgerichteten Zustand des Zielgeräts und ggf. die Richtung und/oder den Betrag der Abweichung von der ausgerichteten Position anzuzeigen.

Ein Ausführungsbeispiel wird anhand der nachfolgenden Figuren näher erläutert.

Es zeigt:
- Fig. 1: die Spitze eines Verriegelungsnagels mit zwei Schwingkreisen,
- Fig. 2: einen Verriegelungsnagel mit zwei Schwingkreisen, von denen einer um eine der Querbohrungen verläuft, und
- Fig. 3: die Ausrichtung der Aufnahme der Schwingkreise relativ zu den Schwingkreisen in dem Verriegelungsnagel.

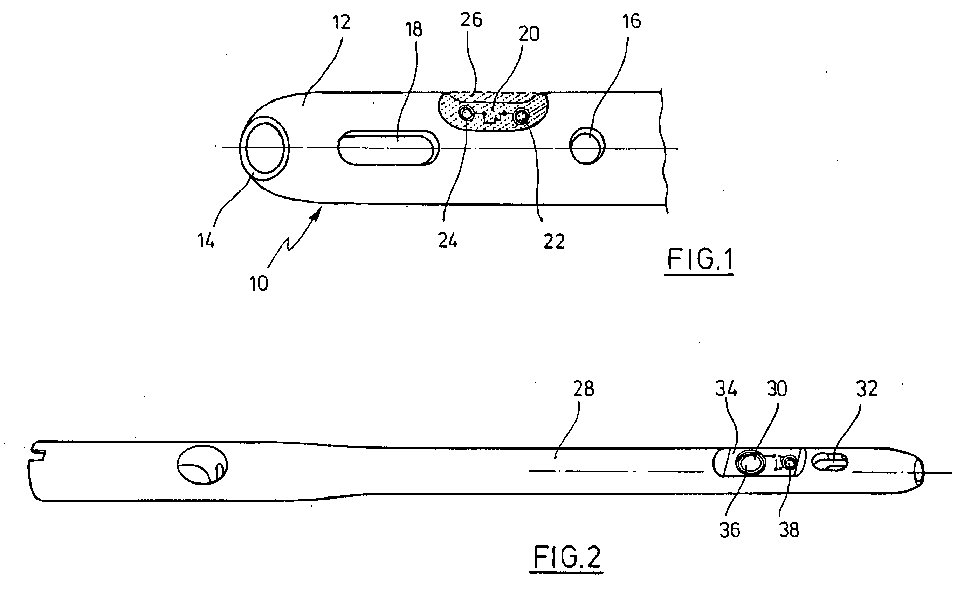

Fig. 1 zeigt die Spitze eines Verriegelungsnagels 10, der einen hohlen Schaft 12 mit einer Öffnung in der Spitze 14 besitzt. In dem Schaft 12 sind zwei Querbohrungen 16 und 18 vorgesehen. Querbohrung 18 ist in dem darstellten Ausführungsbeispiel als ein Langloch ausgeführt. In der Schaftwandung ist im Bereich zwischen den Querbohrungen 16 und 18 eine Ausnehmung 20 in der Schaftwandung eingebracht. In der Ausnehmung 20 sind zwei schematisch dargestellte Schwingkreise 22 und 24 vorgesehen. Die Schwingkreise besitzen jeweils eine Spule und einen Kondensator, die elektrisch aufeinander abgestimmt sind. In dem dargestellten Ausführungsbeispiel sind die Schwingkreise elektrisch miteinander verbunden. Es ist aber auch denkbar, zwei elektrisch unabhängige Schwingkreise vorzusehen. Um die Schwingkreise zum Schwingen anzuregen, erzeugt das Zielgerät einen kurzen Hochfrequenz-Impuls (HF-Impuls). Die Frequenz des Impulses entspricht der Resonanzfrequenz des anzuregenden Schwingkreises im Nagel oder liegt in der Nähe der Resonanzfrequenz, so daß keine zu große Dämpfung in dem Schwingkreis auftritt. Das erzeugte HF-Feld regt den jeweiligen Schwingkreis im Nagel an. Nach Abschaltung des HF-Anregungsfeldes führt die im Schwingkreis gespeicherte Energie dazu, daß der Schwingkreis für die Zeitdauer von einigen Millisekunden weiterschwingt und selbst ein abklingendes HF-Feld erzeugt. Dieses Feld wird vom Zielgerät mit seinen Schwingkreisen empfangen und analysiert. Die Erzeugung des HF-Impulses erfolgt im Zielgerät mit einer großflächigen Spule. Das anregende Feld besitzt im gesamten Zielbereich eine gleichmäßige Feldstärkenverteilung, um unabhängig von der Position des Nagels im Erfassungsgebiet eine gleichbleibend starke Anregung der Schwingkreise im Nagel zu erzielen. Die Anregung der Schwingkreise im Nagel erfolgt somit im Gegensatz zu dem deutschen Gebrauchsmuster DE 202 04 655 drahtlos über einen HF-Impuls. Durch diese Art der Anregung ist es möglich, die im Nagel vorgesehenen Schwingkreise sowohl als Empfangs- als auch Sendeschwingkreise einzusetzen.

Die in der Ausnehmung 20 vorgesehenen Schwingkreise 22, 24 sind durch einen geeigneten, biokompatiblen Kunststoff 26 elektrisch isoliert. Es versteht sich von selbst, daß die Spulen auch gegenüber der Schaftwandung elektrisch isoliert sind.

Fig. 2 zeigt eine alternative Ausgestaltung des Verriegelungsnagels. Bei dieser Ausführungsform besitzt der Verriegelungsnagel 28 im Bereich seiner Spitze zwei Querbohrungen 30 und 32. Querbohrung 32 ist wieder als Langloch ausgebildet. Die Ausnehmung für die zwei Schwingkreise ist im Gegensatz zu dem Ausführungsbeispiel aus Fig. 1 nicht zwischen den Bohrungen angeordnet, sondern um die Querbohrung 30. In der Ausnehmung 34 sind zwei Schwingkreise 36, 38 vorgesehen, wobei Schwingkreis 36 um die Querbohrung 30 verläuft.

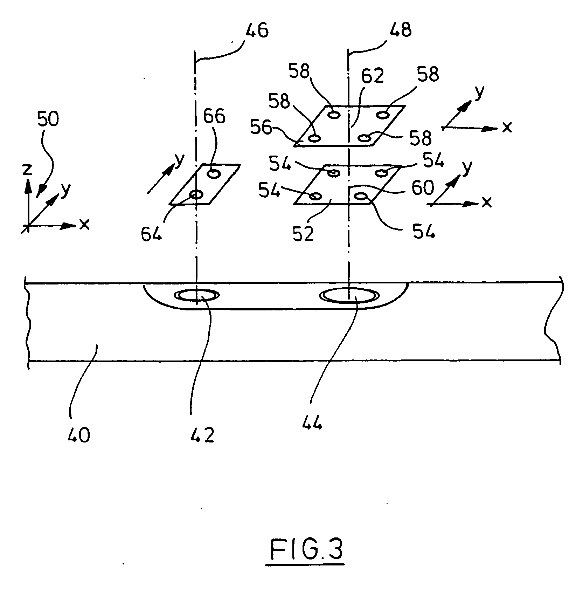

Fig. 3 zeigt in einer schematischen Skizze einen Verriegelungsnagel 40 mit zwei Schwingkreisen 42, 44. Die Spulen der Schwingkreise 42, 44 sind derart in der Schaftwandung angeordnet, daß ihre Hauptabstrahlachsen 46, 48 parallel zueinander verlaufen. Ferner sind die Spulen 42 und 44 entlang der Längsachse des Verriegelungsnagels angeordnet. Zur leichteren Orientierung ist ein Koordinatensystem 50 dargestellt, bei dem die x-Achse entlang der Längsachse des Verriegelungsnagels verläuft und die z-Achse entlang der Hauptabstrahlrichtung 46 und 48. In einer ersten Ebene 52 sind vier Schwingkreise 54 im Quadrat angeordnet. Die erste Ebene 52 verläuft bezogen auf das eingezeichnete Koordinatensystem 50 in der xy-Ebene. Parallel zu der ersten Ebene ist eine zweite Ebene 56 vorgesehen, in der ebenfalls vier Schwingkreise 58 in einem Quadrat zueinander angeordnet sind. Die Ebenen 52 und 56 sind parallel zueinander angeordnet und die Schwingkreise liegen bezogen auf die z-Richtung übereinander. Der Mittelpunkt des Quadrats in der ersten Ebene 52 ist mit 60 gekennzeichnet, während der Mittelpunkt des Quadrats in der zweiten Ebene 56 mit 62 bezeichnet ist. Obwohl die Schwingkreise in dem Ausführungsbeispiel deckungsgleich übereinander dargestellt sind, ist eine solche Anordnung nicht notwendig. Die Schwingkreise können auch zueinander versetzt angeordnet sein.

Fig. 3 zeigt die ausgerichtete Position der ersten Ebene 52 und der zweiten Ebene 56, in der die Hauptabstrahlachse 48 die Zentren 60 und 62 durchstößt.

Messen die Aufnahme-Schwingkreise 54 in der ersten Ebene alle vier ein gleich starkes Signal, so ist das Zielgerät in der xy-Ebene ausgerichtet. Um eine Verkippung zu vermeiden, müssen auch die Schwingkreise 58 gleich starke Signale liefern. Durch diese Bedingung wird sichergestellt, daß sowohl die xy-Ebene getroffen als auch eine Verkippung verhindert ist. Als Freiheitsgrad verbleibt eine Drehung des Zielgeräts um die Achse 48. Um das Zielgerät auch bezüglich dieses Freiheitsgrades festzulegen ist ein zusätzliches Paar von Aufnahmeschwingkreisen 64, 66 vorgesehen. Die Spulen 64 und 66 sind entlang der mit "y" bezeichneten Richtung ausgerichtet und verlaufen jeweils in xy-Richtung. Zeigen auch die Spulen 64, 66 gleich starke Signale an, so ist die Ausrichtung des Zielgeräts bezüglich der Achse 48 festgelegt und eine (nicht dargestellte) Führungshülse weist in Richtung einer der Querbohrungen.

Die Auswerteelektronik beinhaltet zwei Filter- und Verstärkereinheiten, die jeweils auf die Arbeitsfrequenz einer Sendespule im Verriegelungsnagel abgestimmt sind. Die Anbindung der Spuleneinheit geschieht über einen 2x8-Fachmultiplexerbaustein. Der Multiplexer wird von einem Mikrocontroller gesteuert, der bestimmt, welche Meßspule mit der Verstärkereinheit zu verbinden ist. Die Verstärkereinheit besteht aus einem Verstärker und einem Bandpaß. Zur Verbesserung der Störfestigkeit wird der Großteil der Verstärker von einem speziell dimensionierten aktiven Bandpaß durchgeführt. Nach erfolgter Verstärkung und Filterung wird das Signal gleichgerichtet und an einen A-D-Wandler weitergeleitet. Der A-D-Wandler, vorzugsweise 12 Bit, wird, wie auch der Multiplexer, von dem Mikrocontroller gesteuert. Die gewandelten Daten werden dann mit Hilfe des Mikrocontrollers über eine serielle Verbindung an einen PC übertragen. Der PC wertet die Daten aus und stellt die Lage des Zielgeräts graphisch relativ zu den Querbohrungen des Verriegelungsnagels dar.

Wie bereits erwähnt, werden die Spulen 42 und 44 über eine Erregerspule (nicht dargestellt) in dem Zielgerät angeregt. Die Erregerspule ist dabei derart angeordnet, daß die Aufnahmespulen 54, 58, 64 und 66 nicht durch den HF-Impuls gestört werden.

## Patentansprüche

1. Zielgerät für einen Verriegelungsnagel (10; 28) mit zwei Schwingkreisen (22, 24; 36, 38; 42, 44), die entlang der Längsrichtung des Vernegelungsnagels angeordnet sind, das eine Führungshülse zum Ansetzen eines Bohrers und/oder einer Knochenschraube besitzt, die in einem ausgerichteten Zustand des Zielgeräts mit dem Vernegelungsnagel auf eine Querbohrung (16, 18; 30, 32) weist, wobei
- in einer ersten Ebene (52) drei oder mehr Schwingkreise (54) angeordnet sind, die von einem in der Ebene liegenden ersten Zentrum (60) jeweils einen gleichen Abstand besitzen,
- in einer zweiten parallel zu der ersten Ebene verlaufenden Ebene (56) drei oder mehr Schwingkreise (58) angeordnet sind, die von einem in der zweiten Ebene liegenden zweiten Zentrum (62) jeweils einen gleichen Abstand besitzen,
- wobei erste und zweite Ebene (52, 56) derart zueinander angeordnet sind, daß durch das erste und zweite Zentrum eine Flächennormale zu einer der Ebenen verläuft, und
- ein Paar von Schwingkreisen (64, 66) beabstandet von der durch die Zentren (62, 60) verlaufenden Geraden (48) angeordnet ist und deren Verbindungsgerade in dem mit dem Verriegelungsnagel ausgerichteten Zustand im wesentlichen senkrecht zur Längsrichtung des Verriegelungsnagels steht, **dadurch gekennzeichnet, daß** die Verbindungsgerade und die durch die Zentren verlaufende Gerade senkrecht zueinander stehen, daß die Schwingkreise (64, 66) aus dem Paar von Schwingkreisen (64, 66) im ausgerichteten Zustand je auf unterschiedlichen Seiten der Längsrichtung des Verriegelungsnagels angeordnet sind, und daß zusätzlich eine Anregungsspule vorgesehen ist, die einen HF-Impuls zur Anregung der Schwingkreise in dem Verriegelungsnagel aussenden kann.

2. Zielgerät nach Anspruch 1, **dadurch gekennzeichnet, daß** in der ersten Ebene (52) vier Schwingkreise (54) in einem Rechteck zueinander angeordnet sind.

3. Zielgerät nach Anspruch 2, **dadurch gekennzeichnet, daß** die Schwingkreise (54) in einem Quadrat zueinander angeordnet sind.

4. Zielgerät nach einem der Ansprüche 1 bis 3, **dadurch gekennzeichnet, daß** die Schwingkreise (54) in der zweiten Ebene (56) als Rechteck angeordnet sind.

5. Zielgerät nach Anspruch 4, **dadurch gekennzeichnet, daß** die Schwingkreise (58) in einem Quadrat zueinander angeordnet sind.

6. Zielgerät nach einem der Ansprüche 1 bis 5, **dadurch gekennzeichnet, daß** die Schwingkreise in der ersten und der zweiten Ebene (52, 56) deckungsgleich miteinander angeordnet sind.

7. Zielgerät nach einem der Ansprüche 1 bis 6, **dadurch gekennzeichnet, daß** die Schwingkreise mit einer internen Auswerteeinheit verbunden sind.

8. Zielgerät nach einem der Ansprüche 1 bis 6, **dadurch gekennzeichnet, daß** die Schwingkreise mit einer externen Auswerteeinheit verbindbar sind.

9. Zielgerät nach Anspruch 7 oder 8, **dadurch gekennzeichnet, daß** die Auswerteeinheit Filter- und Verstärkungseinheiten aufweist, die auf eine Arbeitsfrequenz der Schwingkreise abgestimmt sind.

10. Zielgerät nach Anspruch 9, **dadurch gekennzeichnet, daß** die Verstärkereinheit einen Verstärker und einen Bandpaß aufweist, wobei die Verstärkung im wesentlichen durch einen aktiven Bandpaß erfolgt.

11. Zielgerät nach Anspruch 10, **dadurch gekennzeichnet, daß** die Auszwerteeinheit einen Gleichrichter und einen H-D-Wandler aufweist, über die verstärkten und gefilterten Meßwerte der Schwingkreise gleichgerichtet und digitalisiert werden können.

12. Zielgerät nach Anspruch 11, **dadurch gekennzeichnet, daß** die Auswerteinheit eine serielle Schnittstelle aufweist, über die die digitalisierten Daten an einen Computer zur Auswertung weitergeleitet werden Können.

13. Zielgerät nach Anspruch 12, **dadurch gekennzeichnet, daß** die Auswerteeinheit einen Computer aufweist, auf dem die ausgewerteten Daten graphisch dargestellt werden können.

## Claims

1. A target device for an intramedullary nail (10; 28) with two oscillation circuits (22, 24; 36, 38; 42, 44) that are arranged along the longitudinal direction of the intramedullary nail, which target device comprises a guide sleeve for positioning a drill and/or a bone screw, that in an aligned state of the target device with the intramedullary nail points to a transverse bore (16, 18; 30, 32), wherein
- in a first plane (52) three or more oscillation circuits (54) are arranged, each being arranged so as to be equidistant from a first centre (60) situated in the plane;
- in a second plane (56), which extends parallel to the first plane, three or more oscillation circuits (58) are arranged, each being arranged so as to be equidistant from a second centre (62) situated in the second plane;
- wherein the first plane (52) and the second plane (56) are arranged in such a manner relative to each other that through the first and the second centre a surface normal extends to one of the planes; and
- a pair of oscillation circuits (64, 66) is arranged so as to be spaced apart from the straight line (48) extending through the centres (62, 60), and the connecting straight line of said oscillation circuits (64, 66) in the state of being aligned with the intramedullary nail is essentially arranged so as to be perpendicular to the longitudinal direction of the intramedullary nail,
**characterised in that**
- the connecting straight line and the straight line extending through the centres are aligned so as to be perpendicular to each other, that each of the oscillation circuits (64, 66) from the pair of oscillation circuits (64, 66) in the aligned state is arranged on different sides of the longitudinal direction of the intramedullary nail, and **in that** in addition an excitation coil is provided that can emit an HF pulse to excite the oscillation circuits in the intramedullary nail.

2. The target device according to claim 1, **characterised in that** in the first plane (52) four oscillation circuits (54) are arranged in a rectangle relative to each other.

3. The target device according to claim 2, **characterised in that** the oscillation circuits (54) are arranged in a square relative to each other.

4. The target device according to any one of claims 1 to 3, **characterised in that** the oscillation circuits (54) in the second plane (56) are arranged as a rectangle.

5. The target device according to claim 4, **characterised in that** the oscillation circuits (58) are arranged in a square relative to each other.

6. The target device according to any one of claims 1 to 5, **characterised in that** the oscillation circuits in the first and the second planes (52, 56) are arranged so as to be congruent with each other.

7. The target device according to any one of claims 1 to 6, **characterised in that** the oscillation circuits are connected to an internal evaluation unit.

8. The target device according to any one of claims 1 to 6, **characterised in that** the oscillation circuits are connectable to an eternal evaluation unit.

9. The target device according to claim 7 or 8, **characterised in that** the evaluation unit comprises filter and amplification units that are attuned to an operating frequency of the oscillation circuits.

10. The target device according to claim 9, **characterised in that** the amplifier unit comprises an amplifier and a band pass, wherein amplification essentially takes place by means of an active band pass.

11. The target device according to claim 10, **characterised in that** the evaluation unit comprises a rectifier and an A-D converter, by way of which the amplified and filtered measured values of the oscillation circuits can be rectified and digitalised.

12. The target device according to claim 11, **characterised in that** the evaluation unit comprises a serial interface by way of which the digitalised data can be conveyed to a computer for evaluation.

13. The target device according to claim 12, **characterised in that** the evaluation unit comprises a computer on which the evaluated data can be graphically displayed.

## Revendications

1. Appareil de visée pour une broche de verrouillage (10 ; 28) comprenant deux circuits oscillants (22, 24 ; 36, 38 ; 42, 44), qui sont disposés le long de la direction longitudinale de la broche de verrouillage, lequel appareil présente une gaine de guidage pour la pose d'un foret et/ou d'une vis osseuse, orientée dans un état d'alignement de l'appareil de visée avec la broche de verrouillage sur un alésage transversal (16, 18 ; 30, 32), appareil dans lequel
- trois circuits oscillants (54) ou plus sont disposés dans un premier plan (52), lesquels circuits présentent chacun une distance égale par rapport à un premier centre (60) situé dans le plan,
- trois circuits oscillants (58) ou plus sont disposés dans un second plan (56) parallèle au premier plan, lesquels circuits présentent chacun une distance égale par rapport à un second centre (62) situé dans le second plan,
- le premier et le second plan (52, 56) sont disposés mutuellement de telle sorte qu'une normale surfacique par rapport à l'un des plans s'étend au travers du premier et du second centre, et
- une paire de circuits oscillants (64, 66) est disposée à distance de la droite (48) passant par les centres (62, 60) et leur droite de jonction, dans l'état d'alignement avec la broche de verrouillage, se situe de façon essentiellement perpendiculaire à la direction longitudinale de la broche de verrouillage,
**caractérisé en ce que** la droite de jonction et la droite passant par les centres sont perpendiculaires entre elles, que les circuits oscillants (64, 66), constitués de la paire de circuits oscillants (64, 66), sont respectivement disposés dans l'état d'alignement de part et d'autre de la direction longitudinale de la broche de verrouillage, et qu'il est prévu en supplément une bobine excitatrice, qui peut émettre une impulsion HF pour exciter des circuits oscillants dans la broche de verrouillage.

2. Appareil de visée suivant la revendication 1, **caractérisé en ce que** quatre circuits oscillants (54) forment un rectangle dans le premier plan (52).

3. Appareil de visée suivant la revendication 2, **caractérisé en ce que** les circuits oscillants (54) forment un carré.

4. Appareil de visée suivant l'une des revendications 1 à 3, **caractérisé en ce que** les circuits oscillants (58) sont disposés en rectangle dans le second plan (56).

5. Appareil de visée suivant la revendication 4, **caractérisé en ce que** les circuits oscillants (58) forment un carré.

6. Appareil de visée suivant l'une des revendications 1 à 5, **caractérisé en ce que** les circuits oscillants sont mutuellement disposés de manière à coïncider dans le premier et dans le second plan (52, 56).

7. Appareil de visée suivant l'une des revendications 1 à 6, **caractérisé en ce que** les circuits oscillants sont reliés à une unité d'évaluation interne.

8. Appareil de visée suivant l'une des revendications 1 à 6, **caractérisé en ce que** les circuits oscillants peuvent être reliés à une unité d'évaluation externe.

9. Appareil de visée suivant l'une des revendications 7 et 8, **caractérisé en ce que** l'unité d'évaluation présente des unités de filtrage et d'amplification, accordées sur une fréquence de travail des circuits oscillants.

10. Appareil de visée suivant la revendication 9, **caractérisé en ce que** l'unité d'amplification présente un amplificateur et un filtre passe-bande, l'amplification s'effectuant essentiellement par un filtre passe-bande actif.

11. Appareil de visée suivant la revendication 10, **caractérisé en ce que** l'unité d'évaluation présente un redresseur et un convertisseur A - D (analogique-digital), par l'intermédiaire desquels les valeurs mesurées après filtrage et amplification des circuits oscillants peuvent être redressées et numérisées.

12. Appareil de visée suivant la revendication 11, **caractérisé en ce que** l'unité d'évaluation présente une interface sérielle, par l'intermédiaire de laquelle les données numérisées peuvent être transmises pour évaluation à un ordinateur.

13. Appareil de visée suivant la revendication 12, **caractérisé en ce que** l'unité d'évaluation présente un ordinateur, sur lequel les données évaluées peuvent être représentées graphiquement.
